# EUROPEAN PATENT SPECIFICATION

(11) **EP 3 313 339 B1**
(45) Date of publication and mention of the grant of the patent: **24.07.2019**
(21) Application number: 16732208.0
(22) Date of filing: 20.06.2016
(51) Int. Cl.: A61F 13/15, B65H 29/12

(54) **METHOD AND APPARATUS FOR SELECTIVELY FOLDING ABSORBENT ARTICLES**
VERFAHREN UND VORRICHTUNGEN ZUM SELEKTIVEN FALTEN SAUGFÄHIGER ARTIKEL
PROCÉDÉ ET APPAREIL POUR LE PLIAGE SÉLECTIF D'ARTICLES ABSORBANTS

(30) Priority: 24.06.2015 US 201562183796 P
(43) Date of publication of application: 02.05.2018
(73) Proprietor: The Procter & Gamble Company, Cincinnati, OH 45202 (US)
(72) Inventor: SCHNEIDER, Uwe, Cincinnati, Ohio 45202 (US)
(74) Representative: Mather, Peter Geoffrey
(86) International application number: PCT/US2016/038298
(87) International publication number: WO 2016/209751

(56) References cited:
- US-A1- 2008 223 537
- US-A1- 2014 342 894

## Description

### FIELD OF THE INVENTION

The present disclosure relates to methods for making absorbent products, and more particularly, methods for selectively folding absorbent articles.

### BACKGROUND OF THE INVENTION

Along an assembly line, various types of articles, such as for example, diapers and other absorbent articles, may be assembled by adding components to and otherwise modifying an advancing, continuous web of material. For example, in some processes, advancing webs of material are combined with other advancing webs of material. In other examples, individual components created from advancing webs of material are combined with advancing webs of material, which in turn, are then combined with other advancing webs of material. Webs of material and component parts used to manufacture diapers may include: backsheets, topsheets, leg cuffs, waist caps, absorbent core components, front and/or back ears, fastener components, and various types of elastic webs and components such as leg elastics, barrier leg cuff elastics, stretch side panels, and waist elastics. Once the desired component parts are assembled, the advancing web(s) and component parts are subjected to a final knife cut to separate the web(s) into discrete diapers or other absorbent articles. After the final knife cut, absorbent articles may also undergo a folding process prior to packaging.

Diaper pants may also be subjected to additional manufacturing steps not used in the manufacture of conventional taped diapers. For example, diaper pants may include a front elastic belt and a rear elastic belt connected with opposing longitudinal end regions of a chassis that includes an absorbent core. Thus, after folding the chassis into a U-shape about a lateral centerline, opposing lateral end regions of the elastic belts may be connected with each other at side seams to form a diaper pant having a waist opening and a pair of leg openings.

In some instances, it may be desirable to manufacture diaper pants having a wide range of sizes on the same converting line. The diaper pants may advance through apparatuses that fold portions of the elastic belts and/or chassis in various ways to provide a relatively compact and neatly folded diaper pant. The folded diaper pants may then advance from the folding operations to a stacking apparatus that collects and arranges the folded diapers into stacks. And from the stacking apparatus, stacks of folded diapers may advance to a packaging apparatus that places stacks of folded diapers into packages.

Operating a converting line adapted to manufacture absorbent articles having a wide range sizes may present challenges with regard to the folding the diaper pants in desired ways prior to packaging. For example, when manufacturing relatively large sized diaper pants, such as adult incontinence products, it may be desirable to fold various components of the diaper pants in some manner to allow for a relatively better fit within a package. However, when manufacturing relatively small sized diaper pants, such as baby diapers, it may not be necessary or desirable to subject the diaper pants to the same folding process as may be desired for relatively large diaper pants. As such, when changing the operation of converting line from manufacturing large diaper pants to small diaper pants, it may be necessary to physically remove and/or replace equipment on the converting line to accommodate different folding operations needed for the different sized diapers. Such removal and/or replacement of equipment may result in delays in manufacturing operations as well as increased capital investments.

Consequently, it would be beneficial to provide methods for folding absorbent articles in different ways without the need to remove and/or replace equipment to accommodate the manufacture of different sized absorbent articles.

### SUMMARY OF THE INVENTION

The present disclosure relates to methods for folding absorbent articles in different ways, wherein the absorbent articles may be in the form of diaper pants. As discussed herein, the folding apparatus may be reconfigurable to operate in different ways so as to selectively subject advancing diaper pants to different folding operations. For example, the folding apparatus may include a reconfigurable side panel folder and/or a reconfigurable chassis folder. In some embodiments, the side panel folder may operate to fold side panels onto exterior surfaces of the advancing diaper pants or may operate to tuck the side panels inside the advancing diaper pants. In some embodiments, the chassis folder may operate to redirect an advancing diaper pant into a nip, thereby creating a fold line across the mid region of the diaper pant or operate to convey a diaper pant past the nip, thereby bypassing the operation of creating a fold line across the mid region of the diaper pant. As such, the folding apparatus may be reconfigurable to fold diaper pants of different sizes in different ways without the need to remove and/or replace equipment.

In one form, a method for selectively folding absorbent articles comprises the steps of: conveying a first absorbent article in a first direction on a first carrier surface, wherein the first absorbent article includes a leading end region and a trailing end region, and a mid region located between the leading and trailing end regions; advancing the leading end region of the first absorbent article past a nip defined between the first carrier surface and a second carrier surface; redirecting the mid region of the first absorbent article into the nip thereby creating a fold line across the mid region of the first absorbent article; conveying the folded first absorbent article in a second direction between the first carrier surface and the second carrier surface; and advancing the folded first absorbent article to a first location downstream of the nip; conveying a second absorbent article in the first direction with the first carrier surface, wherein the second absorbent article includes a leading end region and a trailing end region, and a mid region located between the first and second end regions; advancing the leading end region, mid region, and trailing end region of the second absorbent article past the nip; conveying the second absorbent article in a third direction away from the nip with the second carrier surface; and advancing the second absorbent article to the first location downstream of the nip.

### BRIEF DESCRIPTION OF THE DRAWINGS

Figure 1A is a front perspective view of a diaper pant.
Figure 1B is a rear perspective view of a diaper pant.
Figure 2 is a partially cut away plan view of the diaper pant shown in Figures 1A and 1B in a flat, uncontracted state.
Figure 3A is a cross-sectional view of the diaper pant of Figures 2 and 5 taken along line 3A-3A.
Figure 3B is a cross-sectional view of the diaper pant of Figures 2 and 5 taken along line 3B-3B.
Figure 4 is a front perspective view of a diaper pant constructed with an outer cover.
Figure 5 is a partially cut away plan view of the diaper pant shown in Figure 4 in a flat, uncontracted state.
Figure 6A is a front plan view of a diaper pant.
Figure 6B is a rear plan view of a diaper pant.
Figure 7 is a schematic side view of a reconfigurable folding apparatus.
Figure 8 is a schematic side view of a side panel tucker apparatus.
Figure 8A is a cross-sectional view of a diaper pant and side panel tucker of Figure 8 taken along line 8A-8A.

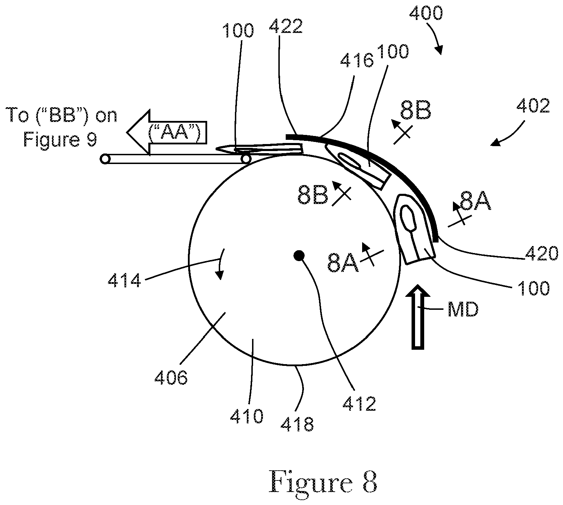

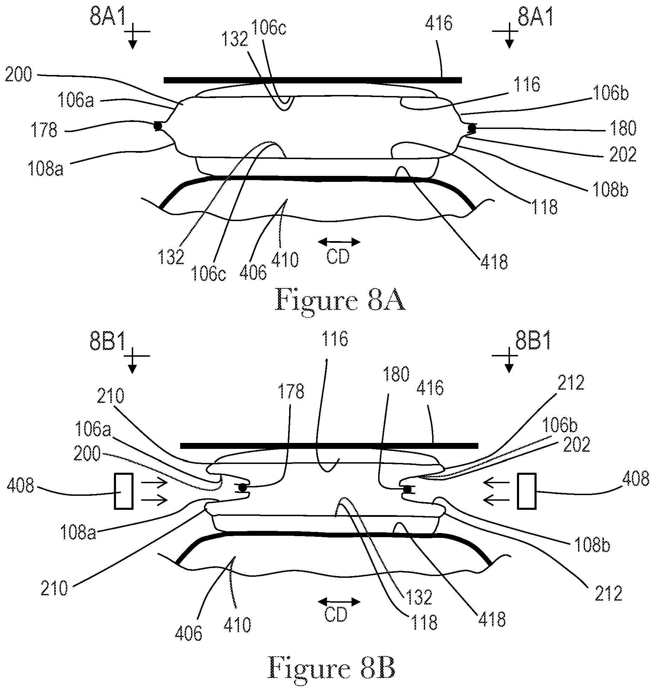

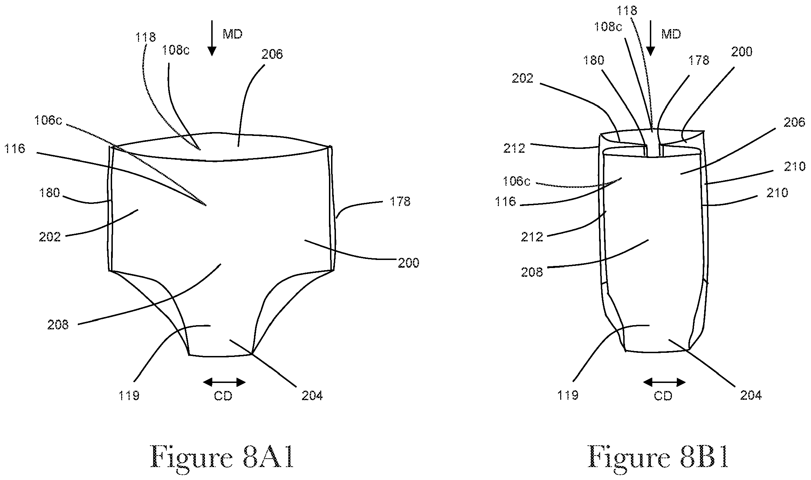

Figure 8A1 is a view of a diaper pant with untucked side panels from Figure 8 A taken along line 8A1-8A1.
Figure 8B is a cross-sectional view of a diaper pant and side panel tucker of Figure 8 taken along line 8B-8B.
Figure 8B1 is a view of a diaper pant with tucked side panels from Figure 8B taken along line 8B1-8B1.

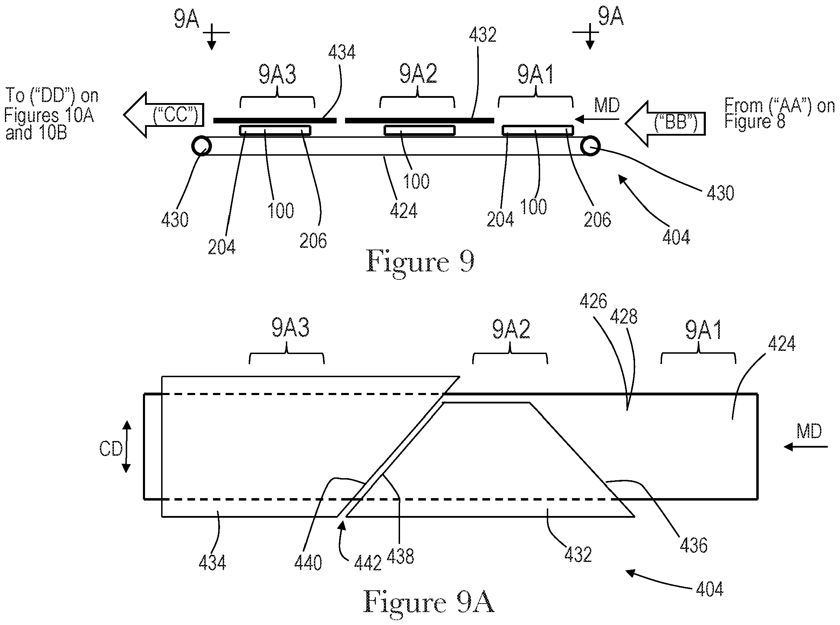

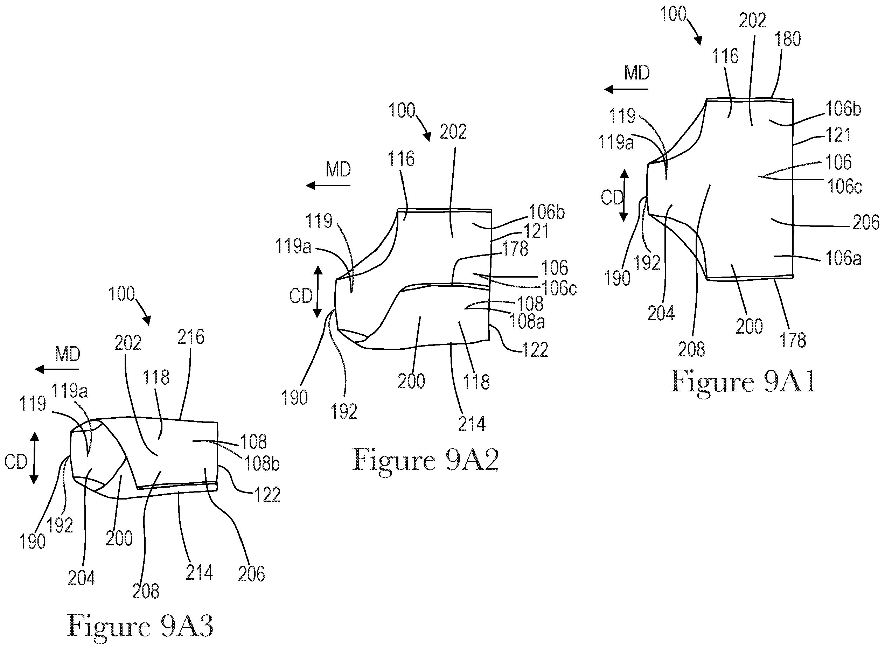

Figure 9 is a schematic side view of an external side folder.
Figure 9A is a view of the external side folder from Figure 9 taken along line 9A-9A.
Figure 9A1 is a view a diaper pant advancing through the external side folder from Figure 9 taken along line 9A-9A at location 9A1.
Figure 9A2 is a view a diaper pant advancing through the external side folding from Figure 9 taken along line 9A-9A at location 9A2.
Figure 9A3 is a view a diaper pant advancing through the external side folder from Figure 9 taken along line 9A-9A at location 9A3.

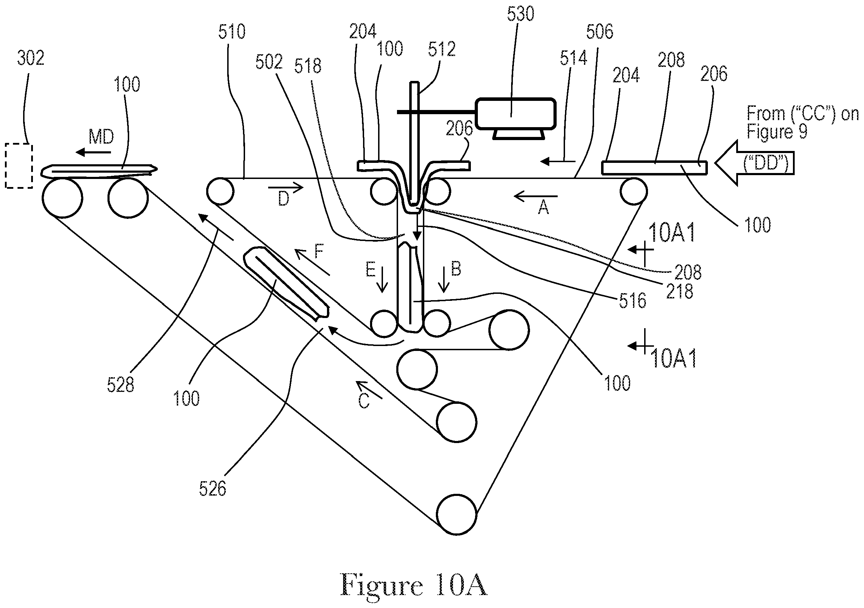

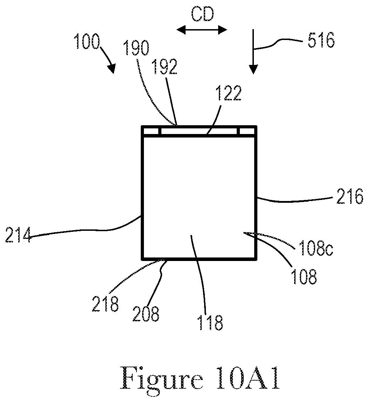

Figure 10A is a schematic side view of a chassis folder operating to redirect an advancing diaper pant into a nip, thereby creating a fold line across the mid region of the diaper pant.
Figure 10A1 is a view a folded diaper pant advancing through the chassis folder from Figure 10A taken along line 10A1-10A1.

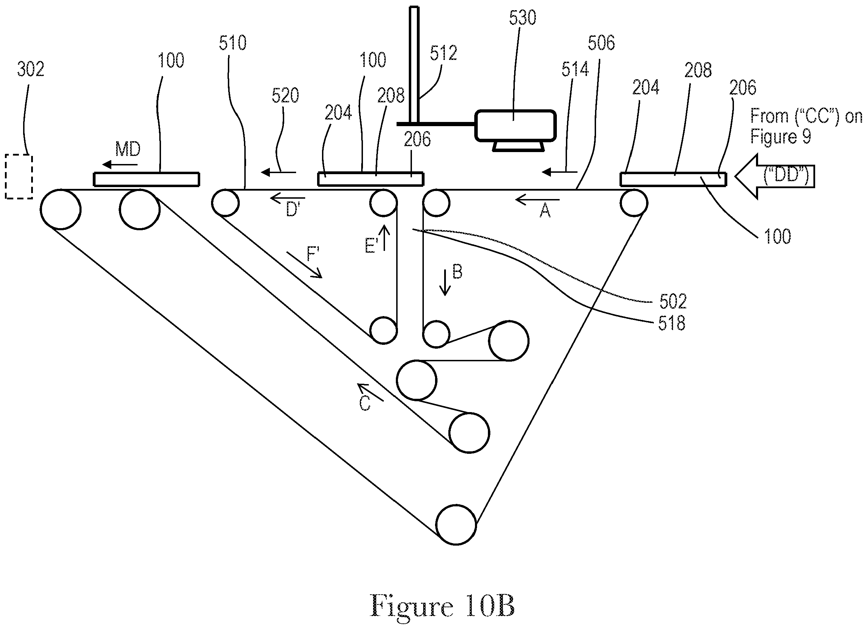

Figure 10B is a schematic side view of a chassis folder operating to convey a diaper pant past the nip, thereby bypassing the operation of creating a fold line across the mid region of the diaper pant.

### DETAILED DESCRIPTION OF THE INVENTION

The following term explanations may be useful in understanding the present disclosure:
"Absorbent article" is used herein to refer to consumer products whose primary function is to absorb and retain soils and wastes. "Diaper" is used herein to refer to an absorbent article generally worn by infants and incontinent persons about the lower torso. The term "disposable" is used herein to describe absorbent articles which generally are not intended to be laundered or otherwise restored or reused as an absorbent article (e.g., they are intended to be discarded after a single use and may also be configured to be recycled, composted or otherwise disposed of in an environmentally compatible manner).
An "elastic," "elastomer" or "elastomeric" refers to materials exhibiting elastic properties, which include any material that upon application of a force to its relaxed, initial length can stretch or elongate to an elongated length more than 10% greater than its initial length and will substantially recover back to about its initial length upon release of the applied force.
As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.
"Longitudinal" means a direction running substantially perpendicular from a waist edge to a longitudinally opposing waist edge of an absorbent article when the article is in a flat out, uncontracted state, or from a waist edge to the bottom of the crotch, i.e., the fold line, in a bi-folded article. Directions within 45 degrees of the longitudinal direction are considered to be "longitudinal." "Lateral" refers to a direction running from a longitudinally extending side edge to a laterally opposing longitudinally extending side edge of an article and generally at a right angle to the longitudinal direction. Directions within 45 degrees of the lateral direction are considered to be "lateral."
The term "substrate" is used herein to describe a material which is primarily two-dimensional (i.e. in an XY plane) and whose thickness (in a Z direction) is relatively small (i.e. 1/10 or less) in comparison to its length (in an X direction) and width (in a Y direction). Nonlimiting examples of substrates include a web, layer or layers or fibrous materials, nonwovens, films and foils such as polymeric films or metallic foils. These materials may be used alone or may comprise two or more layers laminated together. As such, a web is a substrate.
The term "nonwoven" refers herein to a material made from continuous (long) filaments (fibers) and/or discontinuous (short) filaments (fibers) by processes such as spunbonding, meltblowing, carding, and the like. Nonwovens do not have a woven or knitted filament pattern.
The term "machine direction" (MD) is used herein to refer to the direction of material flow through a process. In addition, relative placement and movement of material can be described as flowing in the machine direction through a process from upstream in the process to downstream in the process.
The term "cross direction" (CD) is used herein to refer to a direction that is generally perpendicular to the machine direction.
The term "pant" (also referred to as "training pant", "pre-closed diaper", "diaper pant", "pant diaper", and "pull-on diaper") refers herein to disposable absorbent articles having a continuous perimeter waist opening and continuous perimeter leg openings designed for adult wearers or infant wearers. A pant can be configured with a continuous or closed waist opening and at least one continuous, closed, leg opening prior to the article being applied to the wearer. A pant can be preformed by various techniques including, but not limited to, joining together portions of the article using any refastenable and/or permanent closure member (e.g., seams, heat bonds, pressure welds, adhesives, cohesive bonds, mechanical fasteners, etc.). A pant can be preformed anywhere along the circumference of the article in the waist region (e.g., side fastened or seamed, front waist fastened or seamed, rear waist fastened or seamed).

The present disclosure relates to methods for folding absorbent articles in different ways. As discussed below, the absorbent articles may be in the form of diaper pants, each diaper pant including a first waist region, a second waist region, and a crotch region extending longitudinally between the first waist region and the second waist region. The diaper pant may also include an absorbent chassis extending longitudinally between the first waist region and the second waist region. First and second side panels may extend laterally outward from the first and second waist regions. The first side panel of the first waist region may be connected with the first side panel of the second waist region at a first side seam, and the second side panel of the first waist region may be connected with the second side panel of the second waist region at a second side seam to define a continuous perimeter waist opening and two continuous perimeter leg openings.

In the processes herein, assembled diaper pants may be advanced through a folding apparatus and to a downstream location, such as for example, a stacker apparatus or a packager apparatus. More particularly, assembled diaper pants advance to the folding apparatus in a machine direction such that each diaper pant includes a leading end region and a trailing end region, and a mid region located between the leading and trailing end regions. As the diaper pants advance in the machine direction, the side panels extend laterally outward in a cross direction from either the trailing end region or the leading end region of the advancing diaper pant. As discussed in more detail below, the folding apparatus may be reconfigurable to operate in different ways so as to selectively subject advancing diaper pants to different folding operations. In some embodiments, the folding apparatus may include a reconfigurable side panel folder and/or a reconfigurable chassis folder. For example, in a first configuration, the side panel folder may operate to fold the side panels onto exterior surfaces of the advancing diaper pants. And in a second configuration, the side panel folder may operate to tuck the side panels inside the advancing diaper pants between the first and second waist regions. In another example, in a first configuration, the chassis folder may operate to redirect an advancing diaper pant into a nip, thereby creating a fold line across the mid region of the diaper pant. In a second configuration, the chassis folder may operate to convey a diaper pant past the nip, thereby bypassing the operation of creating a fold line across the mid region of the diaper pant. As such, the folding apparatus may be reconfigurable to fold diaper pants of different sizes in different ways without the need to remove and/or replace equipment.

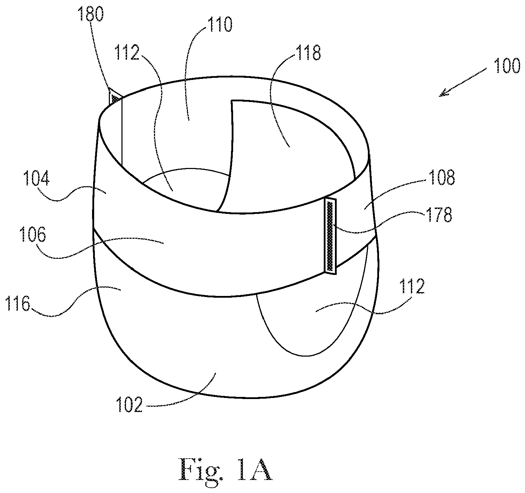

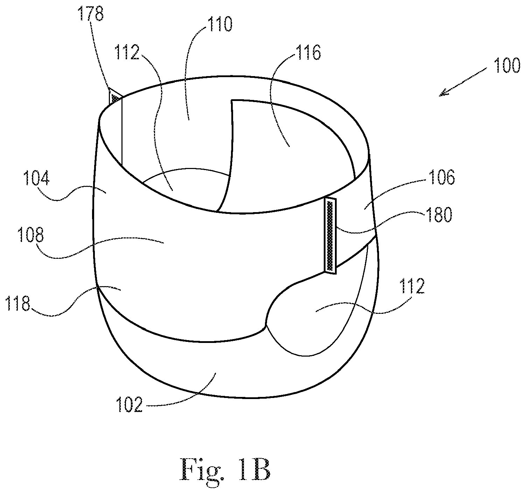

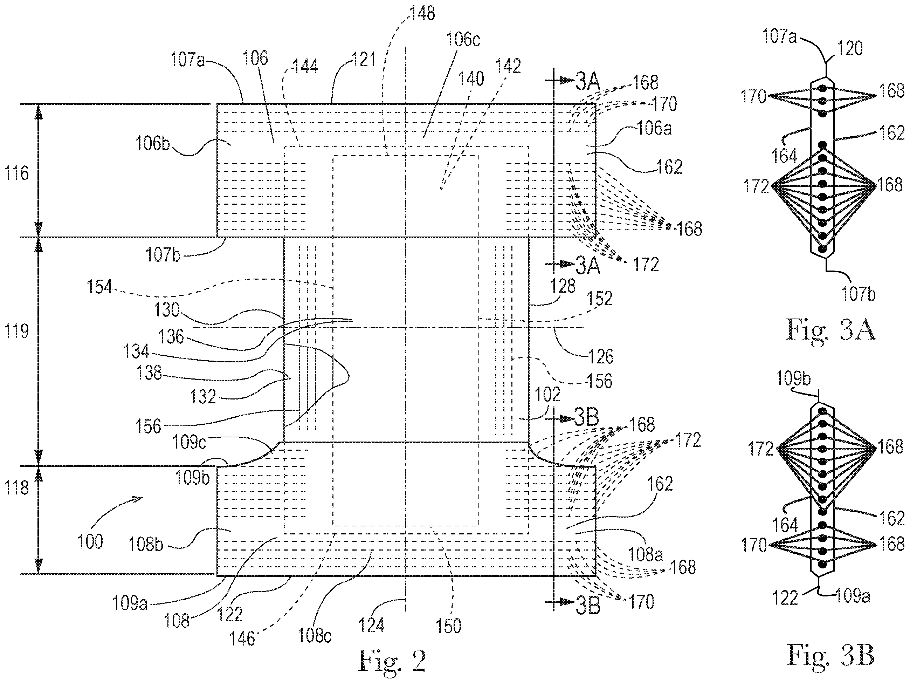

Figures 1A, 1B, and 2 show an example of an absorbent article in the form of a diaper pant 100 that may be assembled and folded in accordance with the methods disclosed herein. In particular, Figures 1A and 1B show perspective views of a diaper pant 100 in a pre-fastened configuration, and Figure 2 shows a plan view of the diaper pant 100 with the portion of the diaper that faces away from a wearer oriented toward the viewer. The diaper pant 100 includes a chassis 102 and a ring-like elastic belt 104. As discussed below in more detail, a first elastic belt 106 and a second elastic belt 108 are bonded together to form the ring-like elastic belt 104. Although only the second elastic belt 108 is shown with a contoured or shaped edge, it is to be appreciated that either or both the first elastic belt 106 and second elastic belt 108 may include contoured edges.

With continued reference to Figure 2, the diaper pant 100 includes a first waist region 116, a second waist region 118, and a crotch region 119 disposed intermediate the first and second waist regions. The first waist region 116 may be configured as a front waist region, and the second waist region 118 may be configured as back waist region. The diaper 100 may also include a laterally extending front waist edge 121 in the front waist region 116 and a longitudinally opposing and laterally extending back waist edge 122 in the back waist region 118. To provide a frame of reference for the present discussion, the diaper 100 and chassis 102 of Figure 2 are shown with a longitudinal axis 124 and a lateral axis 126. In some embodiments, the longitudinal axis 124 may extend through the front waist edge 121 and through the back waist edge 122. And the lateral axis 126 may extend through a first longitudinal or right side edge 128 and through a midpoint of a second longitudinal or left side edge 130 of the chassis 102.

As shown in Figures 1A, 1B, and 2, the diaper pant 100 may include an inner, body facing surface 132, and an outer, garment facing surface 134. The chassis 102 may include a backsheet 136 and a topsheet 138. The chassis 102 may also include an absorbent assembly 140, including an absorbent core 142, disposed between a portion of the topsheet 138 and the backsheet 136. As discussed in more detail below, the diaper 100 may also include other features, such as leg elastics and/or leg cuffs to enhance the fit around the legs of the wearer.

As shown in Figure 2, the periphery of the chassis 102 may be defined by the first longitudinal side edge 128, a second longitudinal side edge 130, a first laterally extending end edge 144 disposed in the first waist region 116, and a second laterally extending end edge 146 disposed in the second waist region 118. Both side edges 128 and 130 extend longitudinally between the first end edge 144 and the second end edge 146. As shown in Figure 2, the laterally extending end edges 144 and 146 are located longitudinally inward from the laterally extending front waist edge 121 in the front waist region 116 and the laterally extending back waist edge 122 in the back waist region 118. When the diaper pant 100 is worn on the lower torso of a wearer, the front waist edge 121 and the back waist edge 122 of the chassis 102 may encircle a portion of the waist of the wearer. At the same time, the chassis side edges 128 and 130 may encircle at least a portion of the legs of the wearer. And the crotch region 119 may be generally positioned between the legs of the wearer with the absorbent core 142 extending from the front waist region 116 through the crotch region 119 to the back waist region 118.

It is to also be appreciated that a portion or the whole of the diaper 100 may also be made laterally extensible. The additional extensibility may help allow the diaper 100 to conform to the body of a wearer during movement by the wearer. The additional extensibility may also help, for example, the user of the diaper 100, including a chassis 102 having a particular size before extension, to extend the front waist region 116, the back waist region 118, or both waist regions of the diaper 100 and/or chassis 102 to provide additional body coverage for wearers of differing size, i.e., to tailor the diaper to an individual wearer. Such extension of the waist region or regions may give the absorbent article a generally hourglass shape, so long as the crotch region is extended to a relatively lesser degree than the waist region or regions, and may impart a tailored appearance to the article when it is worn.

As previously mentioned, the diaper pant 100 may include a backsheet 136. The backsheet 136 may also define the outer surface 134 of the chassis 102. The backsheet 136 may be impervious to fluids (for example, menses, urine, and/or runny feces) and may be manufactured in part from a thin plastic film, although other flexible liquid impervious materials may also be used. The backsheet 136 may prevent the exudates absorbed and contained in the absorbent core from wetting articles which contact the diaper 100, such as bedsheets, pajamas and undergarments. The backsheet 136 may also comprise a woven or nonwoven material, polymeric films such as thermoplastic films of polyethylene or polypropylene, and/or a multilayer or composite materials comprising a film and a nonwoven material (e.g., having an inner film layer and an outer nonwoven layer). The backsheet may also comprise an elastomeric film. An example backsheet 136 may be a polyethylene film having a thickness of from about 0.012 mm (0.5 mils) to about 0.051 mm (2.0 mils). Exemplary polyethylene films are manufactured by Clopay Corporation of Cincinnati, Ohio, under the designation BR-120 and BR-121 and by Tredegar Film Products of Terre Haute, Ind., under the designation XP-39385. The backsheet 136 may also be embossed and/or matte-finished to provide a more clothlike appearance. Further, the backsheet 136 may permit vapors to escape from the absorbent core (i.e., the backsheet is breathable) while still preventing exudates from passing through the backsheet 136. The size of the backsheet 136 may be dictated by the size of the absorbent core 142 and/or particular configuration or size of the diaper 100.

Also described above, the diaper pant 100 may include a topsheet 138. The topsheet 138 may also define all or part of the inner surface 132 of the chassis 102. The topsheet 138 may be compliant, soft feeling, and non-irritating to the wearer's skin. It may be elastically stretchable in one or two directions. Further, the topsheet 138 may be liquid pervious, permitting liquids (e.g., menses, urine, and/or runny feces) to penetrate through its thickness. A topsheet 138 may be manufactured from a wide range of materials such as woven and nonwoven materials; apertured or hydroformed thermoplastic films; apertured nonwovens, porous foams; reticulated foams; reticulated thermoplastic films; and thermoplastic scrims. Woven and nonwoven materials may comprise natural fibers such as wood or cotton fibers; synthetic fibers such as polyester, polypropylene, or polyethylene fibers; or combinations thereof. If the topsheet 138 includes fibers, the fibers may be spunbond, carded, wet-laid, meltblown, hydroentangled, or otherwise processed as is known in the art.

Topsheets 138 may be selected from high loft nonwoven topsheets, apertured film topsheets and apertured nonwoven topsheets. Apertured film topsheets may be pervious to bodily exudates, yet substantially non-absorbent, and have a reduced tendency to allow fluids to pass back through and rewet the wearer's skin. Exemplary apertured films may include those described in U.S. Patent Nos. 5,628,097; 5,916,661; 6,545,197; and 6,107,539.

As mentioned above, the diaper pant 100 may also include an absorbent assembly 140 that is joined to the chassis 102. As shown in Figure 2, the absorbent assembly 140 may have a laterally extending front edge 148 in the front waist region 116 and may have a longitudinally opposing and laterally extending back edge 150 in the back waist region 118. The absorbent assembly may have a longitudinally extending right side edge 152 and may have a laterally opposing and longitudinally extending left side edge 154, both absorbent assembly side edges 152 and 154 may extend longitudinally between the front edge 148 and the back edge 150. The absorbent assembly 140 may additionally include one or more absorbent cores 142 or absorbent core layers. The absorbent core 142 may be at least partially disposed between the topsheet 138 and the backsheet 136 and may be formed in various sizes and shapes that are compatible with the diaper. Exemplary absorbent structures for use as the absorbent core of the present disclosure are described in U.S. Patent Nos. 4,610,678; 4,673,402; 4,888,231; and 4,834,735.

Some absorbent core embodiments may comprise fluid storage cores that contain reduced amounts of cellulosic airfelt material. For instance, such cores may comprise less than about 40%, 30%, 20%, 10%, 5%, or even 1% of cellulosic airfelt material. Such a core may comprises primarily absorbent gelling material in amounts of at least about 60%, 70%, 80%, 85%, 90%, 95%, or even about 100%, where the remainder of the core comprises a microfiber glue (if applicable). Such cores, microfiber glues, and absorbent gelling materials are described in U.S. Patent Nos. 5,599,335; 5,562,646; 5,669,894; and 6,790,798 as well as U.S. Patent Publication Nos. 2004/0158212 and 2004/0097895.

As previously mentioned, the diaper 100 may also include elasticized leg cuffs 156. It is to be appreciated that the leg cuffs 156 can be and are sometimes also referred to as leg bands, side flaps, barrier cuffs, elastic cuffs or gasketing cuffs. The elasticized leg cuffs 156 may be configured in various ways to help reduce the leakage of body exudates in the leg regions. Example leg cuffs 156 may include those described in U.S. Patent Nos. 3,860,003; 4,909,803; 4,695,278; 4,795,454; 4,704,115; 4,909,803; and U.S. Patent Publication Nos. 2009/0312730 A1 and 2013/0255865 A1.

As mentioned above, diaper pants may be manufactured with a ring-like elastic belt 104 and provided to consumers in a configuration wherein the front waist region 116 and the back waist region 118 are connected to each other as packaged, prior to being applied to the wearer. As such, diaper pants may have a continuous perimeter waist opening 110 and continuous perimeter leg openings 112 such as shown in Figures 1A and 1B. The ring-like elastic belt may be formed by joining the first elastic belt to a second elastic belt with a permanent side seam or with an openable and reclosable fastening system disposed at or adjacent the laterally opposing sides of the belts.

As previously mentioned, the ring-like elastic belt 104 is defined by a first elastic belt 106 connected with a second elastic belt 108. As shown in Figure 2, the first elastic belt 106 defines first and second opposing end regions 106a, 106b and a central region 106c, and the second elastic 108 belt defines first and second opposing end regions 108a, 108b and a central region 108c. The central region 106c of the first elastic belt is connected with the first waist region 116 of the chassis 102, and the central region 108c of the second elastic belt 108 is connected with the second waist region 118 of the chassis 102. As shown in Figures 1A and 1B, the first end region 106a of the first elastic belt 106 is connected with the first end region 108a of the second elastic belt 108 at first side seam 178, and the second end region 106b of the first elastic belt 106 is connected with the second end region 108b of the second elastic belt 108 at second side seam 180 to define the ring-like elastic belt 104 as well as the waist opening 110 and leg openings 112.

As shown in Figures 2, 3A, and 3B, the first elastic belt 106 also defines an outer laterally extending edge 107a and an inner laterally extending edge 107b, and the second elastic belt 108 defines an outer laterally extending edge 109a and an inner laterally extending edge 109b. The outer laterally extending edges 107a, 109a may also define the front waist edge 121 and the laterally extending back waist edge 122. The first elastic belt and the second elastic belt may also each include an outer, garment facing layer 162 and an inner, wearer facing layer 164. It is to be appreciated that the first elastic belt 106 and the second elastic belt 108 may comprise the same materials and/or may have the same structure. In some embodiments, the first elastic belt 106 and the second elastic belt may comprise different materials and/or may have different structures. It should also be appreciated that the first elastic belt 106 and the second elastic belt 108 may be constructed from various materials. For example, the first and second belts may be manufactured from materials such as plastic films; apertured plastic films; woven or nonwoven webs of natural materials (e.g., wood or cotton fibers), synthetic fibers (e.g., polyolefins, polyamides, polyester, polyethylene, or polypropylene fibers) or a combination of natural and/or synthetic fibers; or coated woven or nonwoven webs. In some embodiments, the first and second elastic belts include a nonwoven web of synthetic fibers, and may include a stretchable nonwoven. In other embodiments, the first and second elastic belts include an inner hydrophobic, non-stretchable nonwoven material and an outer hydrophobic, non-stretchable nonwoven material.

The first and second elastic belts 106, 108 may also each include belt elastic material interposed between the outer layer 162 and the inner layer 164. The belt elastic material may include one or more elastic elements such as strands, ribbons, films, or panels extending along the lengths of the elastic belts. As shown in Figures 2, 3A, and 3B, the belt elastic material may include a plurality of elastic strands 168 which may be referred to herein as outer, waist elastics 170 and inner, waist elastics 172. Elastic strands 168, such as the outer waist elastics 170, may continuously extend laterally between the first and second opposing end regions 106a, 106b of the first elastic belt 106 and between the first and second opposing end regions 108a, 108b of the second elastic belt 108. In some embodiments, some elastic strands 168, such as the inner waist elastics 172, may be configured with discontinuities in areas, such as for example, where the first and second elastic belts 106, 108 overlap the absorbent assembly 140. In some embodiments, the elastic strands 168 may be disposed at a constant interval in the longitudinal direction. In other embodiments, the elastic strands 168 may be disposed at different intervals in the longitudinal direction. The belt elastic material in a stretched condition may be interposed and joined between the uncontracted outer layer and the uncontracted inner layer. When the belt elastic material is relaxed, the belt elastic material returns to an unstretched condition and contracts the outer layer and the inner layer. The belt elastic material may provide a desired variation of contraction force in the area of the ring-like elastic belt. It is to be appreciated that the chassis 102 and elastic belts 106, 108 may be configured in different ways other than as depicted in Figure 2. The belt elastic material may be joined to the outer and/or inner layers continuously or intermittently along the interface between the belt elastic material and the inner and/or outer belt layers.

In some configurations, the first elastic belt 106 and/or second elastic belt 108 may define curved contours. For example, as shown in Figure 2, the inner lateral edge 109b of the second elastic belt 108 may include non-linear or curved portions 109c in the first and second opposing end regions 108a, 108b. Such curved contours may help define desired shapes to leg opening 112, such as for example, relatively rounded leg openings. Although the inner lateral edge 107b of the first elastic belt is depicted as being a straight line, it is to be appreciated that the inner lateral edge 107b may also include curved portions in the first and second opposing end regions 106a, 106b. In addition to having curved contours, the elastic belts 106, 108 may include elastic strands 168, 172 that extend along non-linear or curved paths that may correspond with the curved contours of the inner lateral edges 107b, 109b.

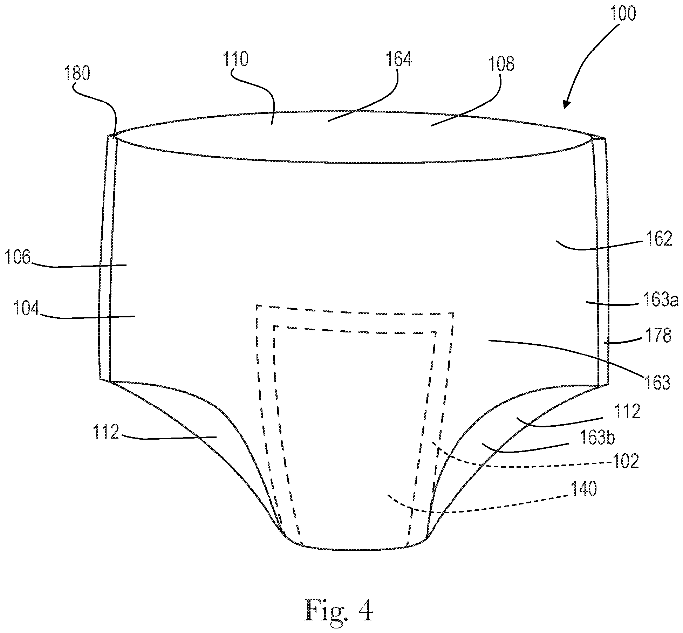

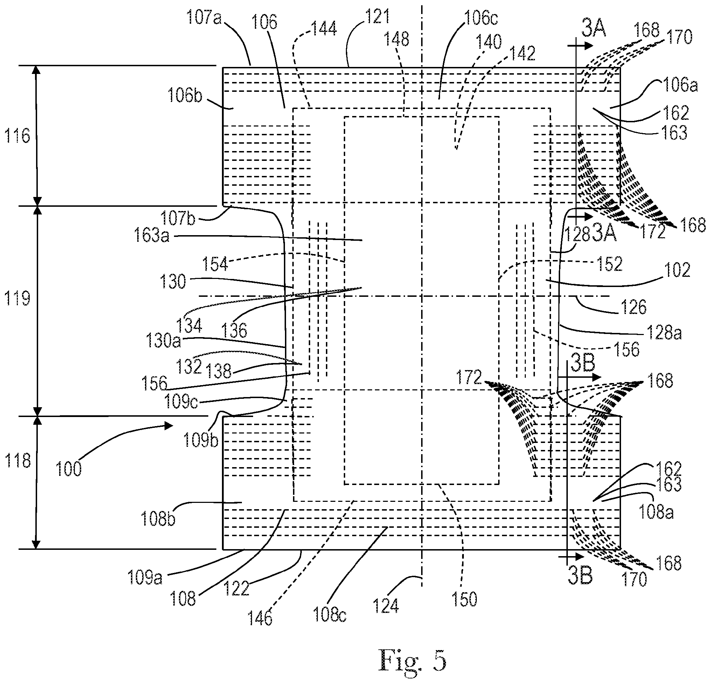

It is to be appreciated that the diaper pant 100 may include a chassis 102 and elastic belts 106, 108 configured in different ways other than as depicted in Figures 1-2B. For example, Figures 4 and 5 show a diaper pant 100 having the same components as described above with reference to Figures 1-2B, except the outer layer 162 of the elastic belt 106, 108 is a configured as a contiguous outer cover 163 that extends through the first waist region 116, crotch region 119, and second waist region 118. Thus, as shown in Figures 4 and 5, the diaper 100 the outer cover 163 also includes a first waist end region 116, a crotch region 119, and an opposing second waist end region 118. The outer cover 163 also includes a garment facing surface 163a and an opposing wearer facing surface 163b. As such, elastic members 168 of the elastic belts 106, 108 may be connected with the wearer facing surface 163b of the outer cover 163. And the chassis 102 may be positioned on the wearer facing surface 163b of the outer cover 163. As such, the backsheet 136 may include a portion of the outer cover 163. In addition, the outer cover 163 may include a first longitudinal side edge 128a and a second longitudinal side edge 130a that are positioned laterally outboard the first longitudinal side edge 128 of the chassis 102 and second longitudinal side edge 130 of the chassis 102, respectively, as shown in Figure 5. It is to be appreciated also that the first longitudinal side edge 128a and a second longitudinal side edge 130a may aligned with or positioned laterally inboard of the first longitudinal side edge 128 of the chassis 102 and second longitudinal side edge 130 of the chassis 102, respectively.

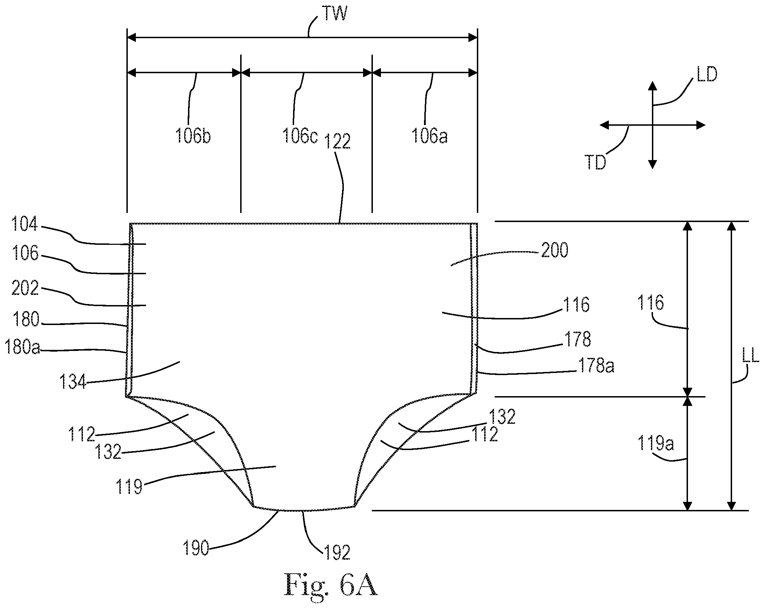

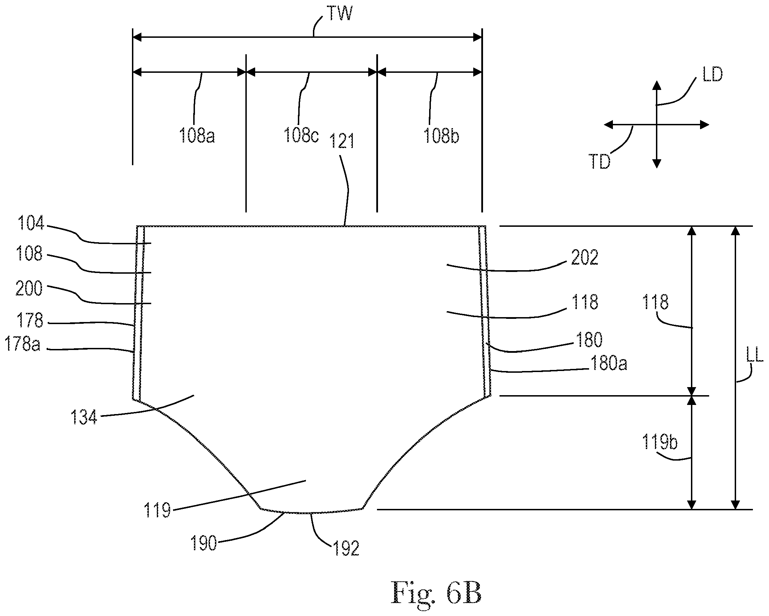

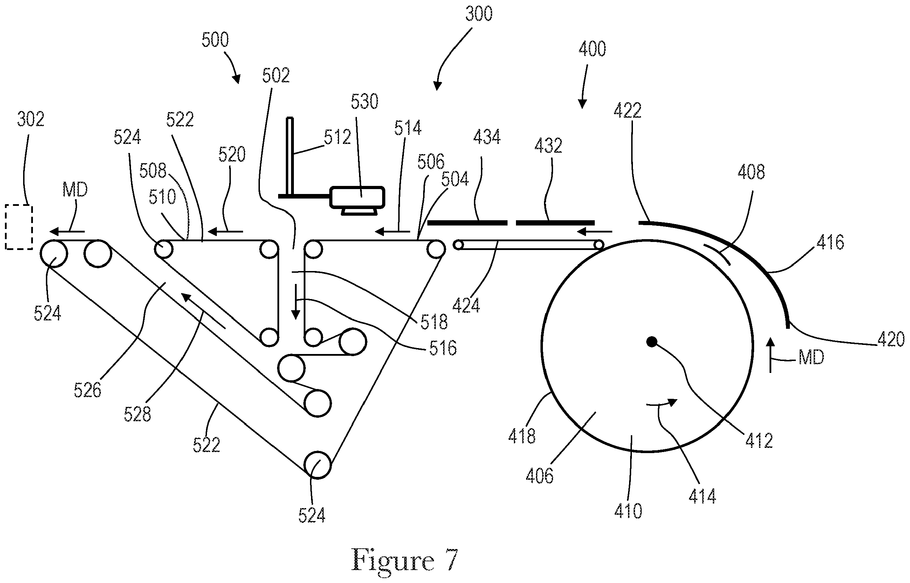

Although the following methods are provided in the context of the diaper pants 100 shown in Figures 1-5, it is to be appreciated that various embodiments of diaper pants can be folded according to the methods disclosed herein, such as for example, the absorbent articles disclosed in U.S. Patent Publication Nos. 2011/0167765 A1, 2013/0130879 A1, and 2013/0211636 A1. As discussed in more detail below with reference to Figures 7-10B, the methods herein may include a folding apparatus 300 adapted to selectively fold absorbent articles 100 and advance the absorbent articles 100 to a first location 302. The absorbent articles may be in the form of diaper pants 100 such as those embodiments discussed above with reference to Figures 1A-5. To help place the following description of the methods in better context as relating to various regions of diaper pants 100 that may be folded, Figures 6A and 6B depict various regions of generic diaper pants 100. In particular, Figure 6A shows a front plan view of a diaper pant 100 in a laid flat condition illustrating various regions of the diaper pant 100. And Figure 6B shows a rear front plan view of diaper pant 100 in a laid flat condition illustrating various regions of the diaper pant 100. As discussed above, the diaper pant 100 defines include an inner, body facing surface 132, and an outer, garment facing surface 134. The diaper pant 100 also defines a longitudinal direction LD that extends from the waist edges 121, 122 to a crotch end 190 that is defined by a lateral fold line 192 in the crotch region 119. As such, the lateral fold line 192 divides the crotch region into a first crotch region 119a and a second crotch region 119b. The diaper pant 100 also defines a transverse direction TD is perpendicular to the longitudinal direction LD.

The diaper pant 100 as shown in Figures 6A and 6B may include a first elastic bell 106 in the first waist region 116, and a second elastic belt 108 in the second waist region 118. As such, a first end region 106a and a second end region 106b extend laterally outward from a central region 106c in the first waist region 116. And a first end region 108a and a second end region 108b extend laterally outward from a central region 108c in the second waist region 118. The first end regions 106a, 108a are connected together at a first side seam 178 to define a first side panel 200. And the second regions are 106b, 108b are connected together at a second side seam 180 to define a second side panel 202. As such, the first and second side panels 200, 202 connect the first waist region 116 with the second waist region 118 of the diaper pant 100. Although depicted in the accompanying figures as being defined by opposing end regions of continuous belts, it is to be appreciated that the side panels 200, 202 may be defined by discrete pieces of material connected with the chassis. In some embodiments, the side panels 200, 202 may be of a single unitary piece construction. The diaper pant also defines a transverse width TW that extends in a transverse direction TD between a first side edge 178a and a second side edge 180a, and is measured along longitudinal midpoints of each side seam 178, 180.

The first end region 106a of the first waist region extends approximately 20% to 40% of the transverse width TW of the diaper pant 100 in an assembled, laid-flat, relaxed condition, and the first end region 108a of the second waist region 118 extends approximately 20% to 40% of the transverse width TW of the diaper pant 100 in an assembled, laid-flat, relaxed condition. The second end region 106b the first waist region 116 extends approximately 20% to 40% of the transverse width TW of the diaper pant 100 in an assembled, laid-flat, relaxed condition, and the second end region 108b the second waist region 118 extends approximately 20% to 40% of the transverse width TW of the diaper pant 100 in an assembled, laid-flat, relaxed condition. The central region 106c the first waist region 116 and extends approximately 20% to 60% of the transverse width TW of the diaper pant 100 in an assembled, laid-flat, relaxed condition, and the central region 108c the second waist region 118 extends approximately 20% to 60% of the transverse width TW of the diaper pant 100 in an assembled, laid-flat, relaxed condition.

The diaper pant 100 in Figures 6A and 6B is also shown as having a longitudinal length LL that is defined by the distance between the first waist edge 121 and the crotch end 190, or if longer, the distance from the second waist edge 122 to the crotch end 190. The longitudinal length LL is measured along the longitudinal centerline 124 of the diaper pant 100. As shown in Figure 6A, the first waist region 116 extends a distance generally in the longitudinal direction LD from the waist edge 121 along the side seams 178, 180 to the leg openings 112, and the second waist region 118 extends a distance generally in the longitudinal direction LD from the waist edge 122 along the side seams 178, 180 to the leg openings 112. Hence, the first crotch region 119a extends a distance from the crotch end 190 to the first waist region 116, and the second crotch region 119b extends a distance from the crotch end 190 to the second waist region 118. In some embodiments, the first waist region 116 and/or the second waist region 118 may extend about two-thirds the longitudinal length LL of the assembled diaper pant 100. In addition, the first crotch region 119a and/or the second crotch region 119b may extend about one-third the longitudinal length LL of the assembled diaper pant 100.

Figure 7 shows a schematic side view of a folding apparatus 300 adapted to fold discrete diaper pants 100 and transfer the folded diaper pants 100 to a first location 302 generically represented by a dashed rectangle. It is to be appreciated that the first location 302 may be various locations on a converting line. For example, the first location 302 may be a stacking apparatus that collects and arranges the folded diapers into stacks, and more particularly, may be a stacker receptacle. In another example, the first location 302 may be a packaging apparatus that places stacks of folded diapers into packages. It is also to be appreciated that the diaper pants 100 may be subjected to various methods and apparatuses of assembly and construction before advancing to the folding apparatus 300. As previously mentioned, the folding apparatus 300 may be reconfigurable to operate in different ways so as to selectively subject advancing diaper pants to different folding operations. As shown in Figure 7, the folding apparatus 300 may include a reconfigurable side panel folder 400 and/or a reconfigurable chassis folder 500. The side panel folder 400 may include a side panel tucker 402 and an external side folder 404. As discussed in more detail below, the side panel folder 400 may be configured such that the side panel tucker 402 operates to tuck side panels 200, 202 inside advancing diaper pants 100 between the first and second waist regions 116, 118. In addition, the side panel folder 400 may be configured such that the external side folder 404 operates to fold the side panels 200, 202 onto exterior surfaces of the advancing diaper pants 100. From the side panel folder, diaper pants 100 may then advance to the chassis folder 500. In turn, the chassis folder 500 may be configured to redirect an advancing diaper pant 100 into a nip 502 between carriers, thereby creating a fold line across the diaper pant 100. In addition, the chassis folder 500 may be configured to bypass the operation of creating a fold line across the diaper pant 100.

As mentioned above with reference to Figures 6A, 6B, and 7, the side panel tucker 402 may be configured to tuck side panels 200, 202 inside advancing diaper pants 100. As shown in Figures 7 and 8, the side panel tucker 402 may include a carrier 406 adapted to convey diaper pants 100 past tucker devices 408 that push the side panels 200, 202 into the chassis 102. As shown in Figure 8, the carrier 406 may be configured as a drum 410 that rotates about an axis of rotation 412 in a direction indicated by arrow 414. The tucker apparatus 402 also includes a guide member 416 positioned radially outward from and along a portion of an outer circumferential surface 418 of the drum 410.

With continued reference to Figures 7 and 8, diaper pants 100 advance in a machine direction MD between the outer surface 418 of the drum 410 and the guide member 416. As shown in Figures 8A and 8B, advancing diaper pants 100 may be oriented such that the second waist region 118 is in contact with the outer surface 418 of the drum 410, and the first waist regions may be in contact with the guide member 416. In addition, as shown in Figures 8A1 and 8B1, the diaper pant 100 may be oriented to advance in the machine direction MD such that the crotch region 119 defines a leading end region 204 and the waist regions 116, 118 define trailing end region 206, with a mid region 208 positioned between the leading end region 204 and the trailing end region 206. The diaper pant 100 may be held onto the outer surface 418 of the drum 410 in various ways, such as with vacuum pressure. As the drum rotates in direction 414, centrifugal forces cause the first waist region 116 to move radially outward from the second waist region 118 and from the outer surface 418 of the drum 410 and toward the guide member 416. As such, the first waist region 116 moves along the guide member 416 from the upstream end region 420 to the downstream end region 422. The guide member 416 is oriented such that a distance between an upstream end region 420 of the guide member 416 and the outer surface 418 of the drum 410 is greater that a distance between a downstream end region 422 of the guide member 416 and the outer surface 418 of the drum 410. As such, the first waist region 116 is forced toward the second waist region 118 as the diaper pant 100 is conveyed from the upstream end region 420 to the downstream end region 422 of the guide member 416.

Before advancing past the downstream end region 422 of the guide member 416, the diaper pants 100 advance past tucker devices 408 that operate to tuck the first and second side panels 200, 202 into the diaper 100 between the first and second waist regions 116, 118. Figures 8A and 8A1 show a diaper pant 100 with the first and second side panels 200, 202 in an untucked configuration, and Figures 8B and 8B1 show the diaper pant 100 with opposing side panels 200, 202 tucked into the chassis 102. As shown in Figure 8B1, portions of each side panel 200, 202 are inserted in between the interior surface 132 of the first waist region 116 and the second waist region 118. In addition, the insertion of the side panels 200, 202 into the chassis 102 defines first longitudinal fold lines 210 along the first waist region 118 and second longitudinal fold lines 212 along the second waist region 118.

It is to be appreciated that the tucker devices 408 may be configured in various different ways. For example, as shown in Figure 8B, the tucker devices 408 are configured as air jets. As the diaper pant 100 advances in the machine direction MD past the tucker devices 408, air discharged in the cross direction, CD, from the air jets impinge on each of the side panels 200, 202 and push the side panels inside the diaper 100 between the waist regions 116, 118. In some embodiments, the tucker devices 408 may be configured as rotating blades that impinge or push on the side panels 200, 202. In yet other embodiments, the tucker devices 408 may be configured as movable rails that converge toward each other in the cross direction CD. It is also to be appreciated that carrier 406 and/or guide member 416 may be configured in various different ways. In some embodiments, the carrier may be configured as a conveyor belt. Additional side panel tucker configurations are disclosed in U.S. Patent Publication No. 2014/0113793 A1 and U.S. Patent Nos. 8,870,732; 9,017,241; 6,723,035; 6,776,316; and 7,270,631.

As previously mentioned, the side panel tucker 402 may be configured to operate to selectively tuck the side panels 200, 202. For example in one configuration as discussed above with reference to Figures 7-8B1, the side panel tucker 402 operates to advance the diaper pants 100 past the tucker devices 408 and tuck the side panels 200, 202 into the diaper between the first and second waist regions. As such, the diaper pants 100 advance from between the drum 410 and the guide member 416 with the inner surfaces 132 of the first and second waist regions 116, 118 in a facing relationship and with the side panels 200, 202 tucked between the first and second waist regions 116, 118. In another example, the side panel tucker 402 may be configured such that the tucker devices 408 do not tuck the side panels 200, 202. For example, the tucker devices may be selectively disabled such that no air is discharged from the tucker devices 408. As such, the diaper pants 100 may advance from the side panel tucker 402 with the inner surfaces 132 of first and second waist regions 116, 118 in a facing relationship and with the side panels 200, 202 untucked.

With reference to Figures 7, 8, and 9, diaper pants 100 may advance from the side panel tucker apparatus 402 to the external side folder 404. As previously mentioned, the external side folder 404 is configured to fold the side panels 200, 202 onto exterior surfaces of the advancing diaper pants 100. As shown in Figure 9, external side folder 404 includes a carrier 424 that advances diaper pants 100 in the machine direction MD. The carrier 424 may be in the form of a belt conveyor including a belt 426 defining a carrier surface 428. As shown in Figure 9, the belt 426 may be routed in an endless loop around rollers 430. As shown in Figures 9 and 9A, the external side folder apparatus 404 also includes a first folding member 432 and a second folding member 434. As discussed in more detail below, the carrier 424 advances discrete diaper pants 100 in the first machine direction MD between the carrier surface 428 and the first and second folding members 432, 434 to fold the first and second side panels 200, 202 along longitudinally extending fold lines.

It is to be appreciated that the external side folder 404 may have various configurations and may fold the side panels 200, 202 in different ways. Examples of such various configurations and side panel folds are disclosed in U.S. Patent No. 5,868,727; U.S. Patent Publication No. 2013/0130879 A1; and U.S. Patent Publication No. 2017/065462 A1.

As shown in Figures 9 and 9A, the first folding member 432 and/or the second folding member 434 may be configured as plates extending along the machine direction MD and separated from the carrier surface 428. The first folding member 432 includes an upstream folding edge 436 and a downstream folding edge 438. And the second folding member 434 includes an upstream folding edge 440. The first folding member 432 and the second folding member 434 may be separated from each other along the machine direction MD to define a slot 442 between the downstream folding edge 438 and the upstream folding edge 440. As shown in Figure 9A, the folding edges 436, 438, 440 may be configured to define acute angles with respect to the machine direction MD, and may extend in a cross direction CD across the carrier surface 428.

As previously mentioned, the side panel tucker 402 may be selectively disabled such that the diaper pants 100 may advance to the external side folder 404 with the inner surfaces 132 of the first and second waist regions 116, 118 in a facing relationship and with the side panels 200, 202 untucked, such as shown in Figure 9A1. Also, as shown in Figure 9A1 and 9A3, the diaper pant 100 may be oriented with respect to the machine direction MD such that the crotch region 119 defines the leading end region 204 and the waist regions 116, 118 define the trailing end region 206, with the mid region 208 positioned between the leading end region 204 and the trailing end region 206. With particular reference to Figures 9, 9A, and 9A1, the diaper pants 100 may be positioned on carrier surface 428 with first waist edge 121 and the second waist edge 122 extending in the cross direction CD and the crotch end 190 positioned downstream of the waist edges 121, 122. In addition, the diaper pant 100 may be positioned on the carrier 424 such that the second waist region 118 and the second crotch region 119b are in a facing relationship with the carrier surface 428. Thus, the carrier 424 advances the diaper pant 100 in the machine direction MD on the carrier surface 428 from location 9A1 and in the orientation shown in Figure 9A1. The diaper pant 100 then advances from location 9A1 to where the first side panel 200 contacts the upstream folding edge 436 of the first folding member 432. As the diaper pant 100 continues to advance in the machine direction MD between the first folding member 432 and the carrier surface 428 to location 9A2, the first folding member 432 folds the first side panel 200 onto the first waist region 116. More particular, the upstream folding edge 436 of the first folding member 432 directs the first end regions 106a, 108a of the first belt 106 and second belt 108 to be folded over the central region 106c of the first belt 106. Thus, as shown in Figure 9A2, the diaper pant 100 is folded along a longitudinally extending first fold line 214 to position the first end region 106a of the first elastic belt 106 in a facing relationship with the central region 106c of the first elastic belt 106.

From position 9A2 on the carrier 424, the diaper pant 100 advances in the machine direction MD to the second folding member 434 and the second side panel 202 is folded onto the first waist region 116 and the folded first side panel 200. More particularly, the second end regions 106b, 108b of the first belt 106 and second belt 108 contact the upstream folding edge 440 of the second folding member 434. As the diaper pant 100 continues to advance in the machine direction MD between the second folding member 434 and the carrier surface 428, the upstream folding edge 440 of the second folding member 434 directs the second end regions 106b, 108b of the first belt 106 and second belt 108 to be folded over the central region 106c of the first belt 106. As such, the second end regions 106b, 108b of the first belt 106 and second belt 108 are directed along the slot 442 between the first and second folding members 432, 434. As shown in the transition from Figure 9A2 to Figure 9A3, the diaper pant 100 is folded along a longitudinally extending second fold line 216 to position the second end region 106b of the first elastic belt 106 in a facing relationship with the first end region 108a of the second elastic belt 108.

As discussed above, the side panel tucker 402 and the external side folder 404 can be reconfigured to operate in different ways so as to selectively subject advancing diaper pants to different folding operations. For example, as discussed above, the side panel tucker 402 can be configured to tuck side panels 200, 202 inside advancing diaper pants 100 between the first and second waist regions 116, 118. As such, a diaper pant 100 with tucked side panels, such as shown in Figure 8B1 may advance from the side panel tucker 402 to the external side folder 404. Because the side panels 200, 202 are tucked inside the diaper 100, the folding members 432, 434 of the external side folder 404 cannot act on the tucked side panels 200, 202. Thus, the diaper pants 100 with tucked side panels 200, 202 may advance to and from the external side folder 404 in the same tucked configuration, such as shown in Figure 8A1. In another example, the side panel tucker 402 can be configured to not tuck side panels 200, 202 inside advancing diaper pants 100. As such, a diaper pant 100 with untucked side panels, such as shown in Figure 9A1 may advance from the side panel tucker 402 to the external side folder 404. Because the side panels 200, 202 are untucked, the folding members 432, 434 of the external side folder 404 act to fold the untucked side panels 200, 202 over external surfaces of the advancing diaper 100. Thus, the diaper pants 100 with folded side panels 200, 202 may advance from the external side folder 404 in a folded configuration, such as shown in Figure 9A3.

As discussed above and with reference to Figure 7, diaper pants 100 may advance from the side panel folder 400 to the chassis folder 500 may be selectively configured to create a fold line across the diaper pant 100. As shown in Figure 7, the chassis folder 500 includes a first carrier 504 having a first carrier surface 506, a second carrier 508 having a second carrier surface 510, and a movable tucker blade 512. The first carrier surface 506 extends in a first machine direction 514 and a second machine direction 516. And the second carrier surface 510 extends in the second machine direction 516 adjacent the first carrier surface 506 to define a nip 502 and a first conveyance path 518 between the first carrier surface 506 and the second carrier surface 510. The first conveyance path 518 extends in the second machine direction 516 between the first and second carrier surfaces 506, 510. The second carrier surface 510 also extends in a third machine direction 520 away from the folding nip 502. With continued reference to Figure 7, the first and/or second carriers 504, 508 may be configured as belt conveyors wherein one or more conveyor belts 522 define the first and/or second carrier surfaces 506, 510. And the belts 522 may be routed in endless loops around rollers 524. As shown in Figure 7, the first and second carriers 504, 508 may also be configured such that a second conveyance path 526 between the first and second carrier surfaces 506, 510 extends in a fourth machine direction 528 away from the first conveyance path 518.

As shown in Figure 7 and as discussed below with reference to Figure 10A, a drive mechanism 530 may be connected with one or more tucker blades 512 to move the tucker blade 512 into contact with the advancing diaper pants 100. It is to be appreciated that the drive mechanism 530 may be configured to impart various types of motion to the tucker blade 512. For example, the drive mechanism 530 may impart a reciprocating motion to the tucker blade 512, such as an up and down (or back and forth) reciprocating motion. In some embodiments, the drive mechanism 530 may be adapted to rotate the tucker blades 512 into engagement with the advancing diaper pants 100. In some configurations, the drive mechanism 530 may include one or more motors directly or indirectly connected with one or more tucker blades 512. In some configurations, a motor may be connected with the tucker blade through various types of transmission, belt, and/or gear arrangements. In addition, the motors may be configured as servo motors that may operate with constant or variable speeds. Various examples of drive mechanism arrangements that may be used to rotate the tucker blades are disclosed in U.S. Patent No. 7,617,656. It is to be appreciated that the drive mechanism may be configured to rotate the tucker blade at a constant angular velocity. In some embodiments, the angular velocity of the tucker blades may be varied on a cyclic basis with a drive mechanism that may include a servo motor. It is to be appreciated that the chassis folder 500 may be configured with various numbers of tucker blades, such as disclosed in for example in U.S. Patent Publication Nos. 2014/0342894 A1 and 2014/0342895 A1.

Referring now to Figures 7, 9, and 10A, diaper pants 100 may advance from the side panel folder apparatus 400 to the chassis folder 500. In one example, the diaper pants 100 may advance from the carrier 424 of the external side folder 404 in a folded configuration, such as shown in Figure 9A3 to the first carrier 504 of the chassis folder 500. Although the carrier 424 and the first carrier 504 are depicted as distinct devices, it is to be appreciated that in some embodiments, the carrier 424 and the first carrier 504 may be the same device. As discussed above, the chassis folder 500 may be selectively operate in a first configuration to create a fold line across the mid region of the diaper pant, and a second configuration, wherein the folding operation is bypassed.

As shown in Figure 10A, the chassis folder 500 may operate in the first configuration with the first carrier surface 506 advancing in directions A, B, and C; wherein direction A is the same as the first machine direction 514; direction B is the same as the second machine direction 516; and direction C is the same as the fourth machine direction 528. And the second carrier surface 510 advances in directions D, E, and F; wherein direction D is the opposite the third machine direction 520; direction E is the same as the second machine direction 516; and direction F is the same as the fourth machine direction 528. As such, the first carrier surface 506 advances the diaper pant 100 in the first machine direction514 and the tucker blade 512 intermittently moves into nip 502. In turn, the leading end region 204 of the absorbent article is advanced past the nip 502 defined between the first carrier surface 506 and the second carrier surface 510. The tucker blade 512 engages the absorbent article 100 and redirects the mid region 208 of the absorbent article 100 into the nip 502 thereby creating a fold line 218 across the mid region 208 of the absorbent article 100, such as shown in Figure 10A1. More particularly, the diaper pants 100 are folded while being forced in the second machine direction 516 and into the nip 502 by the tucker blade 512. For example, from location 9A3 shown in Figure 9, the first carrier 504 conveys the partially folded diaper pants 100 such that the crotch end 190 of each diaper pant 100 advances in the first machine direction 514 past the nip 502 and the tucker blade 512. The tucker blade 512 is moved into position to contact the diaper pant 100 in either the first waist region 116 or crotch region 119 and redirect the diaper pant 100 into the nip 502. As the diaper pant 100 is directed in to the nip 502, the first crotch region 119a is positioned in a facing relationship with the second end region 108b of the second elastic belt 108, thereby creating a laterally extending third fold line 218 to form a folded absorbent article 100, such as shown in Figure 10A1. As the folded articles 100 advance through the nip 502 and the first conveyance path 518, the second waist region 118 is in facing relationship with the first carrier surface 506 and the second crotch region 119b is in a facing relationship with the second carrier surface 510. The folded diaper pant 100 is then advanced in the second machine direction 516 in the first conveyance path 518 between the first carrier surface 506 and the second carrier surface 510. The folded diaper pant 100 is subsequently advanced to the first location 302 downstream of the nip 502 and first conveyance path 518. With continued reference to Figure 10A, the chassis folder 500 may also operate to advance folded diaper pants 100 away from the first conveyance path 518 along the second conveyance path 526 in the fourth machine direction 528.

As mentioned above with reference to Figure 7, the first and/or second carriers 504, 508 may be configured as belt conveyors wherein one or more conveyor belts 522 define the first and/or second carrier surfaces 506, 510. It is also to be appreciated that the first and/or second carriers may be connected with a vacuum pressure source. As such, a vacuum pressure source may be configured to draw air toward the first and/or second carrier surfaces 506, 510 to help hold absorbent articles 100 on the first and/or second carrier surfaces 506, 510. In addition, the second carrier may also be connected with a positive pressure source, such that air may be selectively discharged from the second carrier surface 510 to help prevent the leading end region 204 from being pulled into the nip 502. For example, when in the first mode of operation, as discussed above with reference to Figure 10A, the leading end region 204 of a diaper pant 100 advances past the nip 502 and downstream to the second carrier surface 510. As such, air may be discharged from the second carrier surface 510 and onto the leading end region of the diaper pant 100 to help prevent the leading end region 204 from being pulled into the nip 502 before advancing past the nip 502.

As shown in Figures 7 and 10B, the chassis folder 500 may also operate in the second configuration wherein the tucker blade 512 is prevented from engaging the advancing diaper pants 100, such as by stopping the drive mechanism 530. In addition, the first carrier surface 506 advances in directions A, B, and C, wherein direction A is the same as the first machine direction 514, direction B is the same as the second machine direction 516, and direction C is the same as the fourth machine direction 528. And the second carrier surface 510 advances in opposite directions than in the first configuration. In particular, the second carrier surface 510 advances in directions D', E', and F', wherein direction D' is the same as the third machine direction 520, direction E' is opposite the second machine direction 516, and direction F' is opposite the fourth machine direction 528. As shown in Figure 10B, when operating in the second configuration, the diaper pants 100 are conveyed in the first machine direction 514 on the first carrier surface 506 toward the nip 502. The leading end region 204, the mid region 208, and the trailing end region 206 of the diaper pants 100 all advance past the nip 502 and onto the second carrier surface 510. In turn, the diaper pants 100 are then conveyed in the third machine direction 520 on the second carrier surface to the first location 302 downstream of the nip 502. As such, the chassis folder 500 may be configured to bypass the operation of creating a fold line 218 across the diaper pant 100.

As discussed above with reference to Figures 8-9A, the side panel folder 400 may be configured to operate to tuck side panels 200, 202 inside the diaper pant 100 or to fold side panels 200, 202 onto exterior surfaces of the diaper pant 100. The diaper pants 100 may advance from the side panel folder 404 with side panels 200, 202 tucked as shown in Figure 8B1 or with the side panels 200, 202 untucked and folded as shown in Figure 9A3 to the chassis folder 500. As discussed above with reference to Figures 10A and 10B, the chassis folder 500 may be reconfigurable to selectively fold the diaper pants 100. For example, with reference to Figure 10A, in a first configuration, the chassis folder 500 may be configured to operate such that the second carrier 510 and tucker blade 512 operate to redirect an advancing diaper pant 100 into the nip 502, thereby creating a fold line 218 across the mid region 208 of the diaper pant 100. The folded diaper pant 100 is then subsequently advanced to the first location 302 downstream of the nip 502. In a second configuration, the second carrier 510 and tucker blade 512 operate to convey a diaper pant 100 past the nip 502, thereby bypassing the operation of creating a fold line 218 across the mid region 208 of the diaper pant 100. The diaper pant 100 is then subsequently advanced to the first location 302 downstream of the nip 502. As such, by changing the directions in which the second surface 510 advances as well as enabling or disabling operation of the tucker devices 408 and/or tucker blade 512, the folding apparatus 300 may be reconfigurable to fold diaper pants 100 of different sizes in different ways without the need to remove and/or replace equipment.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A method for selectively folding absorbent articles, the method comprising the steps of:
conveying a first absorbent article (100) in a first direction (514) on a first carrier surface (506), wherein the first absorbent article (100) includes a leading end region (204) and a trailing end region (206), and a mid region (208) located between the leading and trailing end regions (204, 206);
advancing the leading end region (204) of the first absorbent article (100) past a nip (502) defined between the first carrier surface (506) and a second carrier surface (510);
redirecting the mid region (208) of the first absorbent article (100) into the nip (502) thereby creating a fold line (218) across the mid region (208) of the first absorbent article (100);
conveying the folded first absorbent article (100) in a second direction (516) between the first carrier surface (506) and the second carrier surface (510); and
advancing the folded first absorbent article (100) to a first location (302) downstream of the nip (502);
**characterised by** the steps of:
conveying a second absorbent article (100) in the first direction (514) with the first carrier surface (506), wherein the second absorbent article (100) includes a leading end region (204) and a trailing end region (206), and a mid region (208) located between the first and second end regions (204, 206);
advancing the leading end region (204), mid region (208), and trailing end region (206) of the second absorbent article (100) past the nip (502);
conveying the second absorbent article (100) in a third direction (520) away from the nip (502) with the second carrier surface (510); and
advancing the second absorbent article (100) to the first location (302) downstream of the nip (502).

2. The method of claim 1, wherein the step of redirecting the mid region (208) of the first absorbent article (100) into the nip (502) further comprises moving a tucker blade (512) into the mid region (208) of the first absorbent article (100).

3. The method according to any of the preceding claims, wherein the first location (302) is a stacker receptacle.

4. The method according to any of the preceding claims, wherein the step of advancing the folded first absorbent article (100) to a first location (302) downstream of the nip (502) further comprises conveying the folded first absorbent article (100) away from the nip (502) with the first carrier surface (506) and the second carrier surface (510).

5. The method according to any of the preceding claims, wherein the first carrier surface (506) comprises a conveyor belt.

6. The method according to any of the preceding claims, wherein the second carrier surface (510) comprises a conveyor belt.

7. The method according to any of the preceding claims, wherein the first absorbent article comprises: a first waist region (116), a second waist region (118), and a crotch region (119) extending longitudinally between the first waist region (116) and the second waist region (118); wherein the first and second waist regions (116, 118) each comprise a first end region (106a, 108a) and a second end region (106b, 108b) separated by a central region (106c, 108c); and wherein the first end region (106a) of the first waist region (116) is connected with the first end region (108a) of the second waist region (118) at a first side seam (178), and the second end region (106b) of the first waist region (116) is connected with the second end region (108b) of the second waist region (118) at a second side seam (180) to define a continuous perimeter waist opening (110) and two continuous perimeter leg openings (112), and wherein the method further comprises the steps of:
before redirecting the first absorbent article (100) into the nip (502), folding the first absorbent article (100) along a longitudinally extending first fold line (214) to position the first end region (106a) of the first waist region (116) in a facing relationship with the central region (106c) of the first waist region (116); and
before redirecting the first absorbent article (100) into the nip (502), folding the first absorbent article (100) along a longitudinally extending second fold line (216) to position the second end region (106b) of the first waist region (116) in a facing relationship with the first end region (108a) of the second waist region (118).

8. The method according to any of the preceding claims, wherein the second absorbent article comprises: a first waist region (116), a second waist region (118), and a crotch region (119) extending longitudinally between the first waist region (116) and the second waist region (118); wherein the first and second waist regions (116, 118) each comprise a first end region (106a, 108a) and a second end region (106b, 108b) separated by a central region (106c, 108c); and wherein the first end region (106a) of the first waist region (116) is connected with the first end region (108a) of the second waist region (118) at a first side seam (178), and the second end region (106b) of the first waist region (116) is connected with the second end region (108b) of the second waist region (118) at a second side seam (180) to define a continuous perimeter waist opening (110) and two continuous perimeter leg openings (112), and wherein the method further comprises the steps of:
before advancing the second absorbent article (100) past the nip (502), tucking the first and second side seams (178, 180) between the first waist region (116) and the second waist region (118).

9. The method according to any of the preceding claims, further comprising the step of holding the folded first absorbent article (100) and the second absorbent article (100) with vacuum air.

10. The method of claim 9, wherein the step of advancing the leading end region (204) of the first absorbent article (100) past the nip (502) further comprises discharging air from the second carrier surface (510) onto the leading end region (502) of the first absorbent article (100).

## Patentansprüche

1. Verfahren zum selektiven Falten von Absorptionsartikeln, wobei das Verfahren die folgenden Schritte umfasst:
Befördern eines ersten Absorptionsartikels (100) in einer ersten Richtung (514) auf einer ersten Trägeroberfläche (506), wobei der erste Absorptionsartikel (100) einen vorderen Endbereich (204) und einen hinteren Endbereich (206) enthält, und einen mittleren Bereich (208), der sich zwischen dem vorderen und dem hinteren Endbereich (204, 206) befindet;
Vorschieben des vorderen Endbereichs (204) des ersten Absorptionsartikels (100) an einem zwischen der ersten Trägeroberfläche (506) und einer zweiten Trägeroberfläche (510) definierten Spalt (502) vorbei;
Umlenken des mittleren Bereichs (208) des ersten Absorptionsartikels (100) in den Spalt (502), wodurch eine Faltlinie (218) über den mittleren Bereich (208) des ersten Absorptionsartikels (100) hinweg erzeugt wird;
Befördern des gefalteten ersten Absorptionsartikels (100) in einer zweiten Richtung (516) zwischen der ersten Trägeroberfläche (506) und der zweiten Trägeroberfläche (510); und
Vorschieben des gefalteten ersten Absorptionsartikels (100) zu einer ersten Stelle (302), die sich unterhalb des Spalts (502) befindet;
**gekennzeichnet durch** die folgenden Schritte:
Befördern eines zweiten Absorptionsartikels (100) in der ersten Richtung (514) mit der ersten Trägeroberfläche (506), wobei der zweite Absorptionsartikel (100) einen vorderen Endbereich (204) und einen hinteren Endbereich (206) aufweist, und einen mittleren Bereich (208), der sich zwischen dem ersten und zweiten Endbereich (204, 206) befindet;
Vorschieben des vorderen Endbereichs (204), des mittleren Bereichs (208) und des hinteren Endbereichs (206) des zweiten Absorptionsartikels (100) an dem Spalt (502) vorbei;
Befördern des zweiten Absorptionsartikels (100) in einer dritten Richtung (520) weg von dem Spalt (502) mit der zweiten Trägeroberfläche (510); und
Vorschieben des zweiten Absorptionsartikels (100) hin zu der ersten Stelle (302), die sich unterhalb des Spalts (502) befindet.

2. Verfahren nach Anspruch 1, wobei der Schritt des Umlenkens des mittleren Bereichs (208) des ersten Absorptionsartikels (100) in den Spalt (502) ferner das Bewegen einer Falzklinge (512) in den mittleren Bereich (208) des ersten Absorptionsartikels (100) umfasst.

3. Verfahren nach einem der vorstehenden Ansprüche, wobei die erste Stelle (302) ein Stapelaufnahmebehälter ist.

4. Verfahren nach einem der vorstehenden Ansprüche, wobei der Schritt des Vorschiebens der gefalteten ersten Absorptionsartikels (100) zu einer ersten Stelle (302), die sich unterhalb des Spalts (502) befindet, ferner das Befördern des gefalteten ersten Absorptionsartikels (100) weg von dem Spalt (502) mit der ersten Trägeroberfläche (506) und der zweiten Trägeroberfläche (510) umfasst.

5. Verfahren nach einem der vorstehenden Ansprüche, wobei die erste Trägeroberfläche (506) ein Förderband umfasst.

6. Verfahren nach einem der vorstehenden Ansprüche, wobei die zweite Trägeroberfläche (510) ein Förderband umfasst.

7. Verfahren nach einem der vorstehenden Ansprüche, wobei der erste Absorptionsartikel Folgendes umfasst: einen ersten Taillenbereich (116), einen zweiten Taillenbereich (118) und einen Schrittbereich (119), der sich in Längsrichtung zwischen dem ersten Taillenbereich (116) und dem zweiten Taillenbereich (118) erstreckt; wobei der erste und zweite Taillenbereich (116, 118) jeweils einen ersten Endbereich (106a, 108a) und einen zweiten Endbereich (106b, 108b) umfassen, die durch einen Mittelbereich (106c, 108c) getrennt sind; und wobei der erste Endbereich (106a) des ersten Taillenbereichs (116) mit dem ersten Endbereich (108a) des zweiten Taillenbereichs (118) an einer ersten Seitennaht (178) verbunden ist, und der zweite Endbereich (106b) des ersten Taillenbereichs (116) mit dem zweiten Endbereich (108b) des zweiten Taillenbereichs (118) an einer zweiten Seitennaht (180) verbunden ist, um eine ununterbrochene Umfangstaillenöffnung (110) und zwei ununterbrochene Umfangsbeinöffnungen (112) zu bilden, und wobei das Verfahren ferner die folgenden Schritte umfasst:
vor dem Umlenken des ersten Absorptionsartikels (100) in den Spalt (502), Falten des ersten Absorptionsartikels (100) entlang einer sich in Längsrichtung erstreckenden ersten Faltlinie (214), um den ersten Endbereich (106a) des ersten Taillenbereichs (116) in einer einander zugewandten Beziehung mit dem Mittelbereich (106c) des ersten Taillenbereichs (116) zu positionieren; und
vor dem Umlenken des ersten Absorptionsartikels (100) in den Spalt (502), Falten des ersten Absorptionsartikels (100) entlang einer sich in Längsrichtung erstreckenden zweiten Faltlinie (216), um den zweiten Endbereich (106b) des ersten Taillenbereichs (116) in einer einander zugewandten Beziehung mit dem ersten Endbereich (108a) des zweiten Taillenbereichs (118) zu positionieren.

8. Verfahren nach einem der vorstehenden Ansprüche, wobei der zweite Absorptionsartikel Folgendes umfasst: einen ersten Taillenbereich (116), einen zweiten Taillenbereich (118) und einen Schrittbereich (119), der sich in Längsrichtung zwischen dem ersten Taillenbereich (116) und dem zweiten Taillenbereich (118) erstreckt; wobei der erste und der zweite Taillenbereich (116, 118) jeweils einen ersten Endbereich (106a, 108a) und einen zweiten Endbereich (106b, 108b) umfassen, die durch einen Mittelbereich (106c, 108c) getrennt sind; und wobei der erste Endbereich (106a) des ersten Taillenbereichs (116) mit dem ersten Endbereich (108a) des zweiten Taillenbereichs (118) an einer ersten Seitennaht (178) verbunden ist, und der zweite Endbereich (106b) des ersten Taillenbereichs (116) mit dem zweiten Endbereich (108b) des zweiten Taillenbereichs (118) an einer zweiten Seitennaht (180) verbunden ist, um eine ununterbrochene Umfangstaillenöffnung (110) und zwei ununterbrochene Umfangsbeinöffnungen (112) zu bilden, und wobei das Verfahren ferner die folgenden Schritte umfasst:
vor dem Vorschieben des zweiten Absorptionsartikels (100) an dem Spalt (502) vorbei werden die erste und die zweite Seitennaht (178, 180) zwischen dem ersten Taillenbereich (116) und dem zweiten Taillenbereich (118) eingesteckt.

9. Verfahren nach einem der vorstehenden Ansprüche, das ferner den Schritt des Haltens des gefalteten ersten Absorptionsartikels (100) und des zweiten Absorptionsartikels (100) mit Vakuumluft umfasst.

10. Verfahren von Anspruch 9, wobei der Schritt des Vorschiebens des vorderen Endbereichs (204) des ersten Absorptionsartikels (100) an dem Spalt (502) vorbei ferner das Ausstoßen von Luft aus der zweiten Trägeroberfläche (510) auf den vorderen Endbereich (502) des ersten Absorptionsartikels (100) umfasst.

## Revendications

1. Procédé pour plier sélectivement des articles absorbants, le procédé comprenant les étapes consistant à :
transporter un premier article absorbant (100) dans une première direction (514) sur une première surface de support (506), dans lequel le premier article absorbant (100) inclut une région d'extrémité avant (204) et une région d'extrémité arrière (206), et une région intermédiaire (208) située entre les régions d'extrémités avant et arrière (204, 206) ;
l'avancement de la région d'extrémité avant (204) du premier article absorbant (100) au-delà d'une ligne de contact (502) définie entre la première surface de support (506) et une deuxième surface de support (510) ;
le réacheminement de la région intermédiaire (208) du premier article absorbant (100) dans la ligne de contact (502) en créant de ce fait une ligne de pliage (218) à travers la région intermédiaire (208) du premier article absorbant (100) ;
le transport du premier article absorbant plié (100) dans une deuxième direction (516) entre la première surface de support (506) et la deuxième surface de support (510) ; et
l'avancement du premier article absorbant plié (100) vers un premier emplacement (302) en aval de la ligne de contact (502) ;
**caractérisé par** les étapes consistant à :
transporter un deuxième article absorbant (100) dans la première direction (514) avec la première surface de support (506), dans lequel le deuxième article absorbant (100) inclut une région d'extrémité avant (204) et une région d'extrémité arrière (206), et une région intermédiaire (208) située entre les première et deuxième régions d'extrémité (204, 206) ;
faire avancer la région d'extrémité avant (204), la région intermédiaire (208) et la région d'extrémité arrière (206) du deuxième article absorbant (100) au-delà de la ligne de contact (502) ;
transporter le deuxième article absorbant (100) dans une troisième direction (520) à l'écart de la ligne de contact (502) avec la deuxième surface de support (510) ; et
faire avancer le deuxième article absorbant (100) vers le premier emplacement (302) en aval de la ligne de contact (502).

2. Procédé selon la revendication 1, dans lequel l'étape de réacheminement de la région intermédiaire (208) du premier article absorbant (100) dans la ligne de contact (502) comprend en outre le déplacement d'une lame engageante (512) dans la région intermédiaire (208) du premier article absorbant (100).

3. Procédé selon l'une quelconque des revendications précédentes, dans lequel le premier emplacement (302) est un réceptacle d'empileuse.

4. Procédé selon l'une quelconque des revendications précédentes, dans lequel l'étape consistant à faire avancer le premier article absorbant plié (100) vers un premier emplacement (302) en aval de la ligne de contact (502) comprend en outre le transport du premier article absorbant plié (100) à l'écart de la ligne de contact (502) avec la première surface de support (506) et la deuxième surface de support (510).

5. Procédé selon l'une quelconque des revendications précédentes, dans lequel la première surface de support (506) comprend une bande transporteuse.

6. Procédé selon l'une quelconque des revendications précédentes, dans lequel la deuxième surface de support (510) comprend une bande transporteuse.

7. Procédé selon l'une quelconque des revendications précédentes, dans lequel le premier article absorbant comprend : une première région de ceinture (116), une deuxième région de ceinture (118) et une région d'entrejambe (119) s'étendant longitudinalement entre la première région de ceinture (116) et la deuxième région de ceinture (118) ; dans lequel les première et deuxième régions de ceinture (116, 118) comprennent chacune une première région d'extrémité (106a, 108a) et une deuxième région d'extrémité (106b, 108b) séparées par une région centrale (106c, 108c) ; et dans lequel la première région d'extrémité (106a) de la première région de ceinture (116) est raccordée à la première région d'extrémité (108a) de la deuxième région de ceinture (118) au niveau d'une première jonction latérale (178), et la deuxième région d'extrémité (106b) de la première région de ceinture (116) est raccordée à la deuxième région d'extrémité (108b) de la deuxième région de ceinture (118) au niveau d'une deuxième jonction latérale (180) pour définir une ouverture de taille périmétrique continue (110) et deux ouvertures de jambe périmétriques continues (112), et dans lequel le procédé comprend en outre les étapes consistant à :
avant de réacheminer le premier article absorbant (100) dans la ligne de contact (502), plier le premier article absorbant (100) le long d'une première ligne de pliage s'étendant longitudinalement (214) pour positionner la première région d'extrémité (106a) de la première région de ceinture (116) dans une relation faisant face avec la région centrale (106c) de la première région de ceinture (116) ; et
avant de réacheminer le premier article absorbant (100) dans la ligne de contact (502), plier le premier article absorbant (100) le long d'une deuxième ligne de pliage s'étendant longitudinalement (216) pour positionner la deuxième région d'extrémité (106b) de la première région de ceinture (116) dans une relation faisant face avec la première région d'extrémité (108a) de la deuxième région de ceinture (118).

8. Procédé selon l'une quelconque des revendications précédentes, dans lequel le deuxième article absorbant comprend : une première région de ceinture (116), une deuxième région de ceinture (118) et une région d'entrejambe (119) s'étendant longitudinalement entre la première région de ceinture (116) et la deuxième région de ceinture (118) ; dans lequel les première et deuxième régions de ceinture (116, 118) comprennent chacune une première région d'extrémité (106a, 108a) et une deuxième région d'extrémité (106b, 108b) séparées par une région centrale (106c, 108c) ; et dans lequel la première région d'extrémité (106a) de la première région de ceinture (116) est raccordée à la première région d'extrémité (108a) de la deuxième région de ceinture (118) au niveau d'une première jonction latérale (178), et la deuxième région d'extrémité (106b) de la première région de ceinture (116) est raccordée à la deuxième région d'extrémité (108b) de la deuxième région de ceinture (118) au niveau d'une deuxième jonction latérale (180) pour définir une ouverture de taille périmétrique continue (110) et deux ouvertures de jambe périmétriques continues (112), et dans lequel le procédé comprend en outre les étapes consistant à :
avant de faire avancer le deuxième article absorbant (100) au-delà de la ligne de contact (502), rentrer les première et deuxième jonctions latérales (178, 180) entre la première région de ceinture (116) et la deuxième région de ceinture (118).

9. Procédé selon l'une quelconque des revendications précédentes, comprenant en outre l'étape de maintien du premier article absorbant plié (100) et du deuxième article absorbant (100) avec un vide d'air.

10. Procédé selon la revendication 9, dans lequel l'étape consistant à faire avancer la région d'extrémité avant (204) du premier article absorbant (100) au-delà de la ligne de contact (502) comprend en outre la décharge d'air depuis la deuxième surface de support (510) sur la région d'extrémité avant (502) du premier article absorbant (100).
